# EUROPEAN PATENT SPECIFICATION

(11) **EP 4 365 382 B1**
(45) Date of publication and mention of the grant of the patent: **03.06.2026**
(21) Application number: 24165211.4
(22) Date of filing: 29.01.2021
(51) Int. Cl.: A61B 5/20, A61B 5/00, E03D 11/00, G01G 19/52, A61B 10/00

(54) **A TOILET SYSTEM WITH SENSORS FOR MEASURING EXCRETION OUTPUT OF A USER**
TOILETTENSYSTEM MIT SENSOREN ZUR MESSUNG DER AUSSCHEIDUNGSLEISTUNG EINES BENUTZERS
SYSTÈME DE TOILETTES DOTÉ DE CAPTEURS POUR MESURER LE DÉBIT D'EXCRÉTION D'UN UTILISATEUR

(30) Priority: 31.01.2020 DK PA202070063
(43) Date of publication of application: 08.05.2024
(62) Divisional of application: 21704705.9
(73) Proprietor: Measurelet ApS, 1307 Kobenhavn K (DK)
(72) Inventor: BAGGER, Marie Lommer, 2830 Virum (DK); BO SØNDERGAARD SVENDSEN, Morten, 2800 Kongens Lyngby (DK)
(74) Representative: AWA Denmark A/S

(56) References cited:
- JP-A- 2004 278 107
- JP-A- H1 082 783
- US-A1- 2018 087 969
- US-B1- 9 743 903

## Description

### Technical Field

The present disclosure relates to a toilet system comprising a bowl and an outlet, the toilet system being adapted for discerning between types of excretion excreted into the bowl by a user of the toilet system, the toilet system further comprising at least one sensor device configured to capture at least one signal indicative of a quantity of excretion excreted into the bowl by a user of the toilet system, the sensor device comprising a chamber provided on the outlet of the toilet system in a position downstream of a water seal arranged between the bowl and the outlet of the toilet system and adjacent to at least a part of a lower half of the outlet pipe of the toilet system and being adapted to receive a quantity of liquid pressed through a water seal of the toilet system.

As used herein, the term "excretion", when used as a noun, is intended to encompass in principle any waste product, solid or liquid, that is eliminated from a human body, but particularly urine and feces. Likewise, as used herein the term "excretion" and conjugations thereof, when used as a verb, is intended to encompass the action of expelling in principle any waste product, solid or liquid, that is to be eliminated from a human body, such as, e.g., urine, feces, vomit, saliva or mucus.

As used herein, the term "main axis" is intended to refer to an axis parallel with any one of a base, particularly a base line, and a principal axis, particularly a minor axis, of an object, depending on the specific geometrical shape of the object.

As used herein, the term "liquid pressed through a water seal of the toilet system", and in particular "pressed through", is intended to encompass liquid pressed through a water seal of the toilet system irrespective of how, i.e., including not only by an excretion forcing liquid through the water seal, but also overflowing given that it is the force of gravity pressing on the liquid.

### Background Art

Knowledge of the type and quantity of excretion excreted by patients is of great importance within the health sector since healthcare professionals, such as nurses and doctors, use this information for diagnostic purposes and for monitoring the progress of diseases and convalescence in patients. For instance, this information may be of great value in determining the correct treatment for a patient and whether the treatment chosen is effective or needs adjustment.

Toilets adapted for measuring fecal and urinal output of a user are commonly known. However, by far most known solutions are limited to addressing the issue of measuring the quantity of urine excreted into a toilet by a user. This has in the prior art been done both using weight sensors and using pressure sensors.

Within the health sector, particularly on hospitals, it is therefore furthermore known as common practice for healthcare professionals to measure the quantity of feces excreted into a toilet or a bedpan by a patient by physically collecting and weighing the feces. This process is, however, extremely time consuming and cumbersome, and furthermore impractical from a hygiene-related perspective, for users and healthcare professionals alike. Also, the use of bedpans may be unappealing or even humiliating to the patient or user.

Furthermore, US 2018/368818 A1 discloses a toilet device which includes a toilet body that includes a toilet bowl part, a camera that photographs feces discharged and falling into the toilet bowl part in time series and acquires a plurality of still images of the feces, and a fecal properties estimation part that estimates a change in property of the feces from the plurality of still images acquired by the camera. The toilet device estimates the change in the property of the feces from the still images of the feces photographed in time series by the camera.

JP H10 82783 discloses a toilet system with a water sampling port provided at a trap of a toilet bowl wherein trap water can flow into a tank via a pipe. A pipe branched from a middle of the pipe is connected to a discharging socket. The tank is equipped with a pipe for supplying washing water and a pipe for de-aerating. To measure the water amount in the tank, valves are opened, and after a water level in the trap is lowered to a level of the water sampling port, the valve is opened and discharged urine is received in the trap.

JP 20044278107 A discloses a toilet bowl device having a water seal trap communicating the water hollow into which user's waste drops and a drainage pipe has a water meter provided in the joint of the seal water trap and the drainage pipe to measure the flow speed and flowing time of the flowing water and a urine information estimation means estimating a user's urine volume and the urinating speed by the measuring the result of the water volume detector. The water seal trap, or drain socket, comprises a water level sensor, a valve, and an actuator. During measurement, the valve is closed. After completed measurement, the valve is opened, and the water is lead into the sewer. The drain socket is mounted on the sewer, and the valve is mounted in the sewer such as to enable closing off the sewer until measurement of the water amount is completed.

The prior art toilets, however, have the drawback that the measurements performed are not sufficiently precise. Furthermore, the prior art toilets are at best not able to properly discern between fecal and urinal matter when excreted into the bowl of the toilet by a user. Still further, the prior art toilets are not able to assess the quantity of fecal matter when excreted into the bowl of the toilet by a user.

Another drawback by the prior art toilet systems is that a total replacement of an existing toilet system is necessary, which is a cumbersome, time consuming and expensive procedure.

Furthermore, when using a toilet system of the type mentioned by way of introduction to determine the mass or volume of an object put into a container with an overflow, it is not possible to measure the mass of the bowl as it is constant, or the change in mass only corresponds to the volume required to fill the bowl until it overflows.

Also, different events of interest, such as urination and defecation, causes a smaller outflow than e.g. rinsing the bowl by flushing. Likewise, when rinsing the bowl, particulate matter is likely to flow out of the container.

Thus, if one wants to measure the events of interest, a selective capturing method is needed. This may be obtained by means of a toilet system of the type mentioned by way of introduction where a sensor device is attached to the outlet of the toilet system downstream of the water seal, whereby in case of overflow all the water is collected through a hole or opening provided in the outlet pipe.

However, such a toilet system must be carefully designed to provide for obtaining a reliable and precise measurement result. For instance, providing a too small hole may result in clogging if particulate matter is latched in them, eventually obstructing the flow that were intended for measuring. Another challenge is that very small events of interest, can cause liquid intended for measuring to flow around the hole.

It is thus desired to provide a toilet system of the type mentioned by way of introduction alleviating at least some of the above-mentioned and other drawbacks related to the prior art.

### Summary of Invention

It is therefore the object of the disclosure to provide a toilet system of the type mentioned in the introduction with which the quantity of fecal matter, when excreted into the bowl of the toilet system by a user, may be assessed in manner being simple, reliable and precise, also over time.

A further object of the disclosure is to provide such a toilet system with which the sensor device may also be retrofitted to an existing toilet system, already being installed, in a simple and straight forward manner.

The invention is defined by the subject matter of the independent claims. Particular embodiments of the invention are set out in the dependent claims.

The above and other objects are in a first aspect of the disclosure achieved by means of a toilet system comprising a bowl and an outlet, the toilet system being adapted for assessing the quantity of faecal matter, when excreted into the bowl by a user of the toilet system, the sensor device comprising a chamber provided on the outlet of the toilet system in a position downstream of a water seal arranged between the bowl and the outlet of the toilet system, the chamber being arranged adjacent to at least a part of a lower half of the outlet pipe of the toilet system and being adapted to receive a quantity of liquid pressed through a water seal of the toilet system, and at least one through opening provided in the outlet of the toilet system in the position downstream of the water seal at which the chamber is provided such as to allow liquid being forced through the water seal to flow into the chamber, where the at least one through opening is arranged with a main axis extending perpendicular to a longitudinal axis L of the outlet pipe, and where the at least one through opening comprises a shape tapering in an upstream direction.

Thereby, and especially by providing at least one through opening in the outlet pipe, where the at least one through opening is arranged with a main axis extending perpendicular to a longitudinal axis of the outlet pipe and comprise a shape tapering in an upstream direction, a toilet system is provided with which the quantity of fecal matter, when excreted into the bowl of the toilet system by a user, may be assessed in a manner being simple, reliable and precise, also over time especially since the risk of clogging of the plurality of through openings is minimized.

With such a toilet system, the sensor device may also be retrofitted to an existing toilet system already being in use in a simple and straight forward manner by simply providing the chamber on and the at least one through opening in the existing outlet pipe.

Providing the at least one through opening arranged with a main axis extending perpendicular to a longitudinal axis L of the outlet pipe has the further advantage of enabling that the size of the openings, combined with the total flowrate, or pressure, in the outlet pipe, may then be used to determine how much liquid is captured in the chamber for measuring.

It is noted that the quantity of liquid pressed through a water seal of the toilet system may for instance be between 5 ml/min and 1000 ml/min.

In an embodiment, a plurality of through openings are provided and the plurality of through openings comprise a largest size A measured in a direction perpendicular to the longitudinal axis L of the outlet and a largest size B measured in a direction parallel with the longitudinal axis L of the outlet, where neighboring through openings of the plurality of through openings are arranged spaced apart with a shortest distance C measured in a direction perpendicular to the longitudinal axis L of the outlet and a shortest distance D measured in a direction parallel with the longitudinal axis L of the outlet, and where the plurality of through openings are arranged in a pattern fulfilling the relations C < A and D < 2B.

Small openings may clog if particulate matter is latched in them, eventually obstructing the flow that were intended for measuring. Also, in case of very small excretions which in turn force very small amount of liquid through the water seal, liquid intended for measuring may flow around the openings if the spacing between the openings is too large.

The inventors have shown that by arranging a plurality of through openings in a pattern fulfilling the above-mentioned relations these two disadvantages may be overcome. This in turn further enhances the precision and reliability of the measurements performed.

In an embodiment, the at least one through opening comprises a shape tapering and ending in a tip end pointing in an upstream direction.

Thereby, particulate matter is more easily and reliably guided past the openings, whereby the risk of the openings clogging if particulate matter is latched in them, eventually obstructing the flow that were intended for measuring, is lowered considerably or even avoided altogether.

In an embodiment not encompassed by the wording of the claims, the at least one through opening comprises a cross sectional shape being polygonal, ellipsoid, circular, with any one or more of regular, concave, convex or irregular edge(s), or any combination thereof. Examples of polygonal shapes are for instance triangular, rectangular or diamond-shaped. Thus, openings in the form of slits are also feasible.

Openings with such shapes have the advantage of obtaining the above-mentioned advantages while keeping the geometrical shape of the openings simple and easy to manufacture.

In an embodiment, the at least one through opening comprise a largest size A measured in a direction perpendicular to the longitudinal axis L of the outlet and a largest size B measured in a direction parallel with the longitudinal axis L of the outlet, and the size A is between 0,5 mm and 100 mm. Alternatively, or additionally, the size B is between 0,5 mm and 100 mm.

As mentioned, small openings may clog too easily. Large openings, on the other hand, may result in too much particulate matter ending up in the chamber. This in turn may not only clog the chamber but may also render the measurements faulty or even useless due to the contribution to or influence on the measurements from or by the particulate matter stuck in the chamber and may more importantly render it more difficult to empty the chamber after measurements have been finished.

The inventors have shown that by providing the at least one opening with a size within the above-mentioned intervals these two disadvantages, and especially the latter disadvantage, may be overcome. This in turn further enhances the precision and reliability of the measurements performed.

In an embodiment, the plurality of through openings comprise at least five openings.

Thereby a sufficient number of openings is provided to ensure that a sufficient amount of liquid is captured in the chamber, which in turn ensures reliable and precise measurements.

In an embodiment, the sensor device further comprises a screening device arranged upstream of the at least one through opening or extending over the at least one through opening.

Such a screening device has the advantage of contributing to leading particulate matter past the openings such as to avoid that too much particulate matter ends up in the chamber, while still allowing liquid to flow through the opening(s) and into the chamber. This in turn keeps the chamber from clogging and more importantly enables avoiding the measurements becoming faulty or even useless due to the contribution from the particulate matter stuck in the chamber.

In an embodiment, the sensor device further comprises a mass sensor or a weight sensor arranged and configured to monitor a weight of the chamber and to capture at least one signal indicative of a quantity of excretion excreted into the bowl by a user of the toilet system.

Alternatively, or additionally, the sensor device may comprise a flow sensor arranged and configured to monitor a flow of liquid through the chamber and to capture at least one signal indicative of a quantity of excretion excreted into the bowl by a user of the toilet system.

Thereby, a toilet system is provided with which signals indicative of a quantity of excretion excreted into the bowl by a user of the toilet system may be captured and transmitted to analysis in an analysis device in a particularly simple manner.

In an embodiment, the sensor device further comprises a mounting element adapted for enabling mounting the sensor device on an outlet pipe of a toilet system.

Thereby, a toilet system is provided with which the sensor device may be retrofitted to an existing toilet system already being in use in a particularly simple and straight forward manner by simply using the mounting element when mounting the chamber on an existing outlet pipe.

The mounting element may be an element configured for being attached around an outlet pipe. The mounting element may also be a fastener such as a welding or a gluing.

In an embodiment, the chamber further comprises any one of an outlet and a pumping device adapted for pumping the contents of the chamber into the outlet of a toilet system.

Thereby, the chamber may be emptied in a particularly simple and efficient manner. The contents of the chamber may for instance simply be pressed back through the at least one through opening and back into the outlet pipe.

In an embodiment, the toilet system may further comprise a movable device adapted for covering the at least one through opening, preferably from below, i.e. on the side facing the chamber, and a device for controlling the movable device and the flushing system of the toilet system in such a manner that flushing is delayed until the movable device is brought to cover the at least one through opening.

In an embodiment, the sensor device further comprises a valve mounted in connection with at least one of the one or more openings and configured to control inflow of liquid into the chamber. The valve may comprise an actuator.

Such a valve has the advantage of contributing to leading particulate matter past the openings such as to avoid that too much particulate matter ends up in the chamber, while still allowing liquid to flow through the opening(s) and into the chamber. Such a valve may furthermore be used to accurately control the size of particulate matter allowed to flow past it into the chamber. This in turn keeps the chamber from clogging and more importantly enables avoiding the measurements becoming faulty or even useless due to the contribution from the particulate matter stuck in the chamber.

In an embodiment, especially an embodiment where the sensor device comprises a valve, the sensor device comprises only one opening.

In an embodiment, the sensor device is further configured to transmit the at least one signal indicative of a quantity of excretion excreted into the bowl by a user of the toilet system to a data analysis device.

In an embodiment, the toilet system further comprises an image sensor.

The above and other objects are in a second aspect of the disclosure achieved by means of a method of providing a toilet system adapted for assessing the quantity of faecal matter, when excreted into the bowl by a user of the toilet system, the toilet system comprising at least one sensor device configured to capture at least one signal indicative of a quantity of excretion excreted into the bowl by a user of the toilet system, the excretion being in the form of any waste product, solid or liquid, that is eliminated from a human body, the method comprising the steps of:
- providing at least one through opening in at least a part of a lower half of the outlet of the toilet system in a position downstream of a water seal arranged between the bowl and the outlet of the toilet system such as to allow liquid being forced through the water seal to flow through the openings and into the chamber, where

the at least one through opening is arranged with a main axis extending perpendicular to a longitudinal axis L of the outlet pipe, and where
the at least one through opening comprises a shape including a shape tapering in an upstream direction,
   - providing a chamber adapted to receive a quantity of liquid pressed through a water seal of the toilet system, and
   - arranging the chamber on the outlet of the toilet system in the position downstream of the water seal arranged between the bowl and the outlet of the toilet system and adjacent to at least a part of a lower half of the outlet pipe of the toilet system.

In further embodiments the method further comprises one or more of the further steps:
a) Providing a plurality of through openings to comprise a largest size A measured in a direction perpendicular to the longitudinal axis L of the outlet and a largest size B measured in a direction parallel with the longitudinal axis L of the outlet, and where neighboring through openings are arranged spaced apart with a smallest distance C measured in a direction perpendicular to the longitudinal axis L of the outlet and a smallest distance D measured in a direction parallel with the longitudinal axis L of the outlet, and wherein the plurality of openings are arranged in a pattern fulfilling the relations C < A and D < 2B.
b) In an embodiment not encompassed by the wording of the claims, providing the at least one through opening or the plurality of through openings to comprise a cross sectional shape being polygonal, ellipsoid, circular, with any one or more of a regular, concave, convex or irregular edge, or any combination thereof.
c) Providing the at least one through opening or the plurality of through openings to comprise a largest size A measured in a direction perpendicular to the longitudinal axis L of the outlet and a largest size B measured in a direction parallel with the longitudinal axis L of the outlet, where the size A is between 0,5 mm and 100 mm, and alternatively, or additionally, where the size B is between 0,5 mm and 100 mm.
d) Providing the plurality of through openings to comprise at least five openings.
e) Providing any one or more of:
   - a screening device arranged upstream of the at least one through opening or the plurality of through openings,
   - a mass sensor or a weight sensor arranged and configured to monitor a weight of the chamber and to capture at least one signal indicative of a quantity of excretion excreted into the bowl by a user of the toilet system,
   - a flow sensor arranged and configured to monitor a flow of liquid through the chamber and to capture at least one signal indicative of a quantity of excretion excreted into the bowl by a user of the toilet system, and
   - a mounting element adapted for enabling mounting the sensor device on an outlet pipe of a toilet system.

It is noted that the invention relates to all possible combinations of features recited in the claims.

### Brief Description of Drawings

In the following description embodiments of the disclosure will be described with reference to the schematic drawings, in which
Fig. 1 is a schematic cross-sectional side view of a toilet system comprising a sensor device with a chamber.
Fig. 2 is a schematic cross-sectional side view of a section of an outlet pipe of a toilet system provided with a sensor device comprising a plurality of through openings, of which only one is visible, and a chamber.
Fig. 3 is a schematic cross-sectional side view similar to that of Fig. 2 showing a toilet system with an alternative embodiment of the chamber.
Figs. 4A to 4C schematically illustrate different shapes and patterns of a plurality of through openings of a sensor device of a toilet system.
Figs. 5A and 5B schematically illustrate sizes and distance requirements of a plurality of through openings of a sensor device of a toilet system.
Fig. 6 is a schematic cross-sectional view of an outlet pipe of a toilet system provided with a sensor device comprising a plurality of through openings, of which only one is visible, a chamber and a mounting element.
Fig. 7 is a schematic cross-sectional side view similar to those of Figs. 2 and 3 and showing a toilet system with an alternative embodiment of the sensor device.

### Description of Embodiments

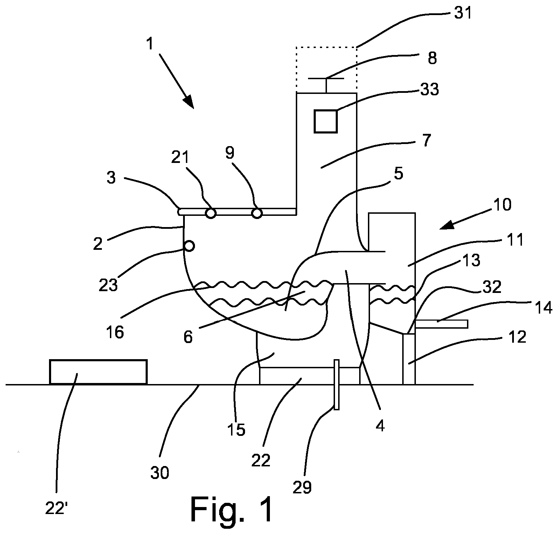

Referring initially to Fig. 1, an embodiment of a toilet system is shown.

The toilet system 1 comprises a bowl 2, an outlet 4 and a seat 3. The outlet 4 further comprises an outlet pipe 5 with a water seal, water trap or U-bend 6. The toilet system 1 may further comprise a toilet system tank or flushing cistern 7 with a device 8 adapted for causing the toilet system to flush when operated. The device 8 may be an actuator such as a press button or a pull button or a lever. The toilet system 1 may further comprise a basis 15 for connection of the toilet system 1 to a floor 30.

The toilet system 1 comprises a sensor device 10 configured to capture a signal indicative of a quantity of excretion excreted into the bowl 2 by a user of the toilet system 1 and an optional sensor device 9 configured to capture a signal indicative of a type of excretion excreted into the bowl 2 by a user of the toilet system 1. The toilet system 1 may also optionally comprise further sensor devices 21, 22, 23.

The toilet system 1 may be a new separate toilet system 1. Alternatively, the sensor devices 9 and 10 may be mounted on an existing toilet system such as to provide a retrofitted toilet system 1. A toilet system 1 may thus be provided by retrofitting an existing toilet system by providing a sensor device 9 and a sensor device 10 and mounting the sensor devices 9 and 10 on the existing toilet system. The at least one through opening or the plurality of through openings to be described further below may be provided in the existing outlet pipe. It is noted that in the context of the present disclosure, the sensor device 9 is optional. It is furthermore noted that the position and type of toilet system 1 may influence on the retrofitting possibilities. For instance, toilet systems with a P-type trap and toilet systems of the wall-hung type may easily be retrofitted.

Generally, the optional sensor device 9 is configured to capture a signal indicative of a type of excretion excreted into the bowl 2 by a user of the toilet system 1. The sensor device is further configured to transmit the captured signal to an analysis device.

The sensor device 9 may be adapted for measuring sound. The sensor device 9 may be a vibration sensor, such as an acoustic sensor or a microphone. Suitable types of microphones include dynamic microphones, piezoelectric microphones, crystal microphones, fiber-optic microphones and MEMS microphones. The sensor device 9 may be wired or wireless.

The sensor device 9 is arranged in or on the seat 3. Alternatively, the sensor device 9 may be arranged in the bowl 2 or on an inner surface of the bowl 2. In any event the sensor device 9 is arranged in a position enabling the sensor device 9 to capture the sounds caused by or in connection with a user excreting an excretion, e.g. voiding his or her bowel and/or bladder, into the bowl 2. The sensor device 9 is arranged above the upper water level 16 in the bowl but may alternatively also be arranged below the upper water level 16 in the bowl.

The at least one sensor device 9 may also comprise or be any one or more of a pressure sensor, a radar, an image capturing device, a distance sensor, a LIDAR, an acoustic distance sensor, and a flow rate sensor, such sensors also being capable of providing a signal indicative of a type of excretion excreted into the bowl 2 by a user of the toilet system 1.

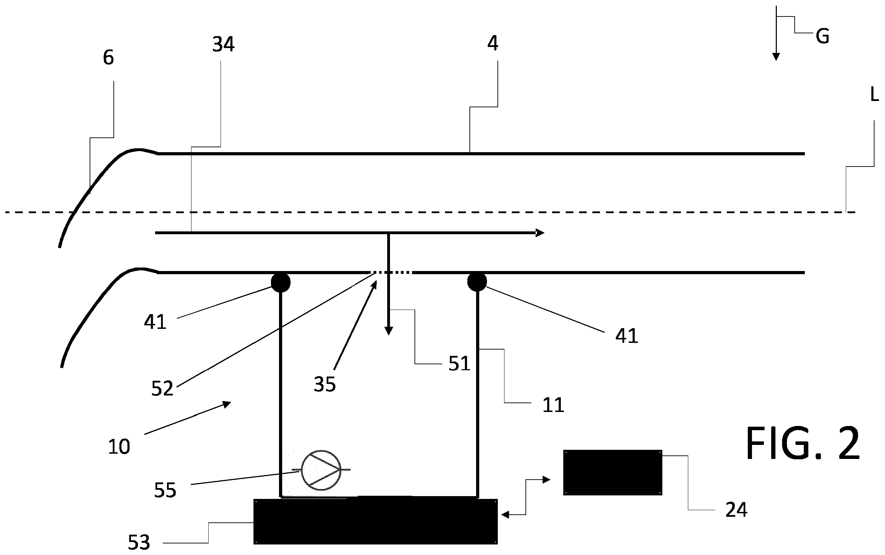

Generally, the sensor device 10 is configured to measure a volume of liquid indicative of a quantity of excretion excreted into the bowl 2 by a user of the toilet system 1. The sensor device 10 may further be configured to transmit the captured signal to an analysis device 24 (Fig. 2). The sensor device 10 may be arranged and configured to measure the amount of liquid forced through the water seal or U-bend 6 when a user voids his or her bowel and/or bladder into the bowl 2. In virtue of Archimedes' law, the amount of liquid forced through the water seal or U-bend 6 when a user voids his or her bowel and/or bladder into the bowl 2 is equivalent, or at least close to equivalent depending on the buoyancy of the excreted material, to the quantity of excretion excreted into the bowl 2 of the user.

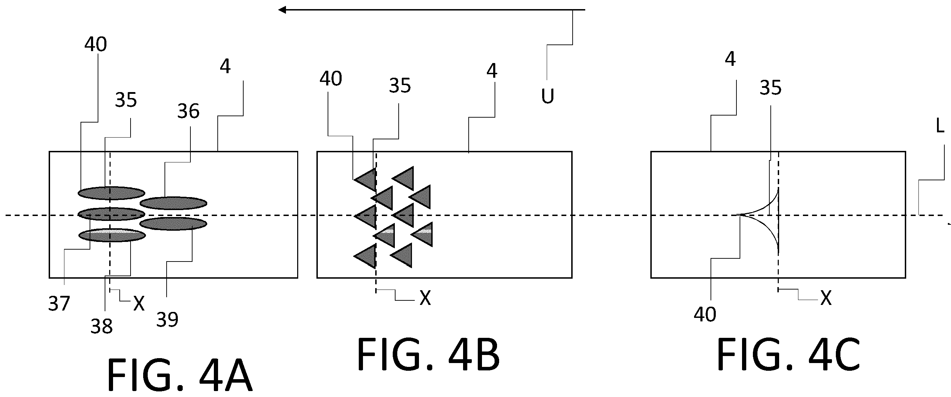

Generally, and referring also to Figs. 2, 3 and 4A-4B the sensor device 10 comprises at least a chamber 11 and a plurality of through openings 35, 36, 37, 38, 39. Alternatively, and referring to Fig. 4C, the sensor device may also comprise at least a chamber 11 and one through opening 35. Generally, the sensor device 10 is arranged in the outlet 4 of the toilet system 1 in a position downstream of the water seal 6.

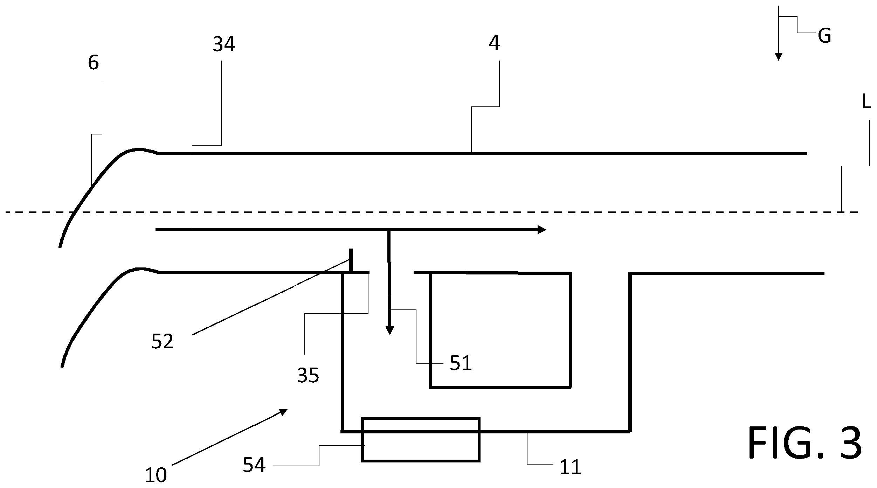

The at least one through opening 35 or the plurality of through openings 35, 36, 37, 38, 39 are provided in the outlet 4 of the toilet system 1 in a position downstream of a water seal 6 arranged between the bowl 2 and the outlet 4 of the toilet system 1, particularly at a lower half of the outlet 4 of the toilet system 1, such as to allow liquid being forced through the water seal 6 to flow into the chamber 11. This may be seen from Figs. 2 and 3, in which the direction of flow 34 in the outlet pipe 4 is shown, and revealing that a part of the water seal 6 may be seen at the left hand side of Fig, 2. The direction of flow 51 of the liquid diverted through the openings 35, 36, 37, 38, 39 into the chamber 11 is also shown on Figs. 2 and 3.

The chamber 11 is provided for collecting the water 13 (Fig. 1) or liquid forced through the water seal 6 as a result of a user depositing an excretion in the bowl 2 of the toilet system 1. The chamber 11 is arranged on the outlet 4 of the toilet system 1 in essentially the same position downstream of the water seal 6 as the plurality of through openings 35, 36, 37, 38, 39. The chamber 11 is, just as the plurality of through openings 35, 36, 37, 38, 39, further arranged adjacent to a lower half of the outlet pipe 4 of the toilet system 1. Thereby, the water 13 forced through the water seal 6 as a result of a user depositing an excretion in the bowl 2 of the toilet system 1 may enter the chamber 11 by the action of gravity. The direction of gravity is indicated on Figs. 2 and 3 by the arrow G.

As shown on Fig. 2, the chamber 11 may be a receptacle. The sensor device 10 may in such an embodiment further comprise a mass sensor or a weight sensor 53 arranged and configured to monitor a weight of the chamber and to capture at least one signal indicative of a quantity of excretion excreted into the bowl by a user of the toilet system. Alternatively, or additionally the sensor device 10 may further comprise a flow sensor, such as e.g. an acoustic flow sensor arranged and configured to monitor a weight of the chamber and to capture at least one signal indicative of a quantity of excretion excreted into the bowl by a user of the toilet system.

Alternatively, and as shown on Fig. 3, the chamber 11 may be a pipe shaped chamber 11 having one end connected to the outlet pipe 4 at the plurality of through openings 35, 36, 37, 38, 39 and another end connected to the outlet pipe 4 at a suitable position further downstream in order to lead the contents of the chamber 11 back into the outlet pipe 4. The sensor device 10 may in such an embodiment further comprise a flow sensor 54 arranged and configured to monitor a weight of the chamber and to capture at least one signal indicative of a quantity of excretion excreted into the bowl by a user of the toilet system. The flow sensor 54 may e.g. be an acoustic flow sensor providing the advantage of enabling monitoring the flow through the chamber 11 without the flow sensor having to be placed in the chamber 11 potentially obstructing the flow to some extent. Alternatively, or additionally the sensor device 10 may further comprise a mass sensor or a weight sensor arranged and configured to monitor a weight of the chamber and to capture at least one signal indicative of a quantity of excretion excreted into the bowl by a user of the toilet system.

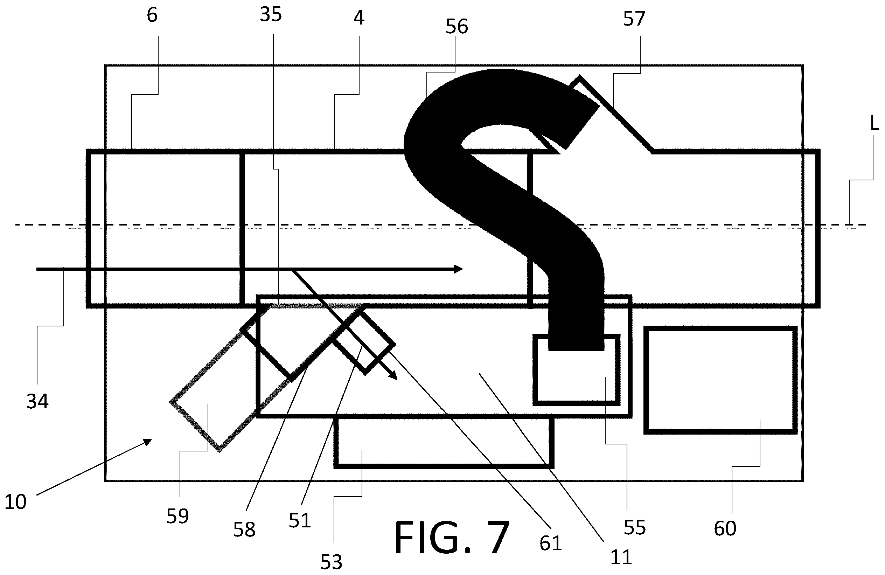

The chamber 11 may furthermore optionally be provided with a pump 55 for evacuating the contents of the chamber 11 back into the outlet pipe 4 - cf. Fig. 7.

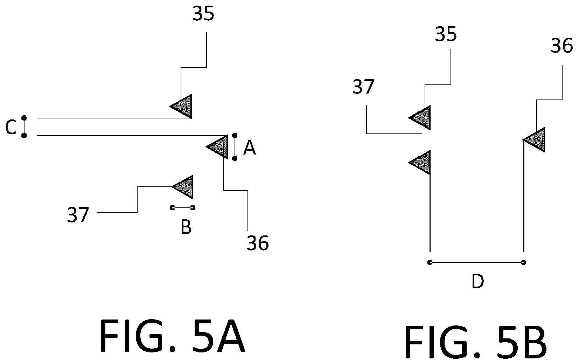

Generally, and referring now specifically to Figs. 4A-4C and 5A-5B, the plurality of through openings 35, 36, 37, 38, 39 are arranged perpendicular to a longitudinal axis L of the outlet pipe 4, particularly with a main axis X arranged extending perpendicular to a longitudinal axis L of the outlet pipe 4. The plurality of through openings 35, 36, 37, 38, 39 each comprise a shape including a tip end 40 (cf. Figs. 4A-4C) pointing in an upstream direction U, i.e. in a direction towards the water seal 6 of the toilet system 1, or in a direction opposite to the direction of flow 34.

The plurality of through openings 35, 36, 37, 38, 39 each comprise a size A (cf. Fig. 5A) measured in a direction perpendicular to the longitudinal axis L of the outlet and a size B (cf. Fig. 5A) measured in a direction parallel with the longitudinal axis L of the outlet. Neighboring through openings are arranged spaced apart with a distance C (cf. Fig. 5A) measured in a direction perpendicular to the longitudinal axis L of the outlet and a distance D (cf. Fig. 5B) measured in a direction parallel with the longitudinal axis L of the outlet. The plurality of openings 35, 36, 37, 38, 39 are arranged in a pattern fulfilling the relations C < A and D < 2B.

The plurality of through openings 35, 36, 37, 38, 39 may comprise a cross sectional shape being triangular (cf. Fig. 4B), oval (cf. Fig. 4A), diamond-shaped or any combination thereof. In case of a triangular opening, the main axis X corresponds to a base of the triangle. In case of an oval or ellipsoid opening, the main axis X corresponds to a minor axis of the oval or ellipsoid. Fig. 4C illustrate another example of an opening 35 being triangular with a straight base and concave sides. The plurality of through openings 35, 36, 37, 38, 39 may further be dimensioned such that the size A is between 0,5 mm and 100 mm and/or such that the size B is between 0,5 mm and 100 mm.

Generally, any number of through openings 35, 36, 37, 38, 39 may be provided. In particular, the number of through openings 35, 36, 37, 38, 39 is five or more, such as six or eight.

The sensor device 10 may further comprise a screening device 52. The screening device 52 may as shown on Fig. 2 be arranged extending over the through openings 35, 36, 37, 38, 39, e.g. as a grating with holes sized to allow liquid, but not solids particles above a predetermined size to pass. Alternatively, the screening device 52 may as shown on Fig. 3 be arranged in a position upstream of the through openings 35, 36, 37, 38, 39. The screening device 52 diverts solids such as feces to flow around the through openings 35, 36, 37, 38, 39 and thereby serves to hinder solids such as feces in falling into the through openings 35, 36, 37, 38, 39 and potentially blocking them. The screening device 52 is furthermore constructed to allow liquids, such as water, to flow around the screening device 52 and fall through the through openings 35, 36, 37, 38, 39 into the chamber 11, while still allowing solids, such as feces, to be led past the through openings 35, 36, 37, 38, 39.

The sensor device 10, or at least the chamber 11 of the sensor device 10, may be configured to be attached to, such as in one piece with, the outlet pipe 4 of the toilet system 1. The sensor device 10, or at least the chamber 11 of the sensor device 10, may also be configured as a pipe section to be attached to and form an integral part of the outlet pipe 4 of the toilet system 1. The plurality of openings 35, 36, 37, 38, 39 may be provided in the existing outlet pipe 4 of the toilet system 1. The plurality of openings 35, 36, 37, 38, 39 may also be provided in the pipe section to be attached to the existing outlet pipe 4 of the toilet system 1.

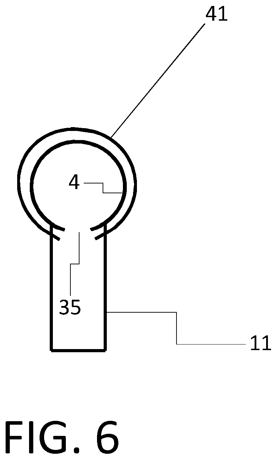

Referring now also to Fig. 6, the sensor device 10 may further comprise a mounting element 41 adapted for enabling mounting the chamber 11 of the sensor device 10 on the outlet pipe 4 of the toilet system 1.

The mounting element 41 may be a welding, a gluing or the like as indicated on Fig. 2. The mounting element 41 may also generally comprise a semi-ring-shaped element configured to extend around and be attached to and/ or tightened to the outlet pipe 4 as shown on Fig. 6.

The mounting element 41 may also be a flexible element being integrally connected to the chamber 11 with at least a first end and being adapted for being wrapped around the outlet pipe 4 and attached with a second end to the chamber 11, for instance in a manner like a terminal strip. In such an embodiment, the connection between the second end of the mounting element 41 and the chamber 11 may for instance be a snap-locking or friction-locking connection.

Referring again to Fig. 1, the sensor device 10 may further comprise a measuring unit 14 configured to measure the quantity of liquid 13 collected in the chamber 11. The sensor device 10 may further comprise an outlet 12 arranged in a bottom area of the chamber 11 and connected to a sewer system or the like. The sensor device 10 may still further comprise a closure mechanism 32, such as a valve, a flap, or a shutter, configured to be closed while the measuring unit 14 measures the quantity of liquid 13 collected in the chamber 11 and to be opened subsequently to allow the chamber 11 to be emptied and the toilet system 1 to be flushed when the toilet system 1 is flushed.

Referring now to Fig. 7, a schematic cross-sectional side view similar to those of Figs. 2 and 3 and illustrating a toilet system with an alternative embodiment of the sensor device 10 is shown. The embodiment shown on Fig. 7 differs from those of Figs. 2 and 3 in virtue of the following features.

The sensor device 10 comprises a pump 55 for evacuating the contents of the chamber 11 back into the outlet pipe 4. The pump 55 may be mounted inside the chamber 11 or outside the chamber and connected to the chamber 11. The pump 55 may be connected to a pipe or hose 56, which in turn may be connected to an inlet 57 leading to the outlet 4 of the toilet system 1 at a position downstream of the at least one opening 35.

The sensor device 10 comprises in this embodiment only one opening 35 provided in the outlet 4 of the toilet system. At or in connection with the opening 35 is provided a valve 58. The valve 58 is provided on the side of the opening 35 facing the chamber 11, and thus outside of the outlet 4. The valve 58 may be of a standard type or it may be custom made. The valve 58 comprises an actuator 59 and an outlet 61. The outlet 61 opens or debouches into the chamber 11, such that liquid lead through the opening 35 is lead through the valve 58 and its outlet 61 into the chamber 11 (flow 51). The actuator 59 may for instance be a motion sensor or a contact sensor which actuates the valve, e.g. upon registering the motion or contact of a liquid flow. The valve 58 may be configured to have a filtering tolerance such as to only let through particles of a suitably small size, such as below 10 mm or below 5 mm or below 0,5 mm. The valve 58 may further be configured to have a maximum capacity enabling the valve 58 to handle a predetermined maximum flow or volume of liquid, for instance corresponding to an expected or statistically feasible maximum volume of an excretion.

It is noted that in embodiments where the sensor device 10 comprises more than one opening 35-39, each such opening 35-39 may comprise an associated valve 58 as described above. However, embodiments of the sensor device 10 comprising a valve 58 generally comprises only one opening 35.

The sensor device 10 according to Fig. 7 further comprises a data processing device 61. The data processing device 61 may have one or more different functions. The data processing device 61 may transmit measurement data detected by the sensor device 53, 54 to a data analysis device. The data processing device 61 may control the actuator 59 of the valve 58. The data processing device 61 may control the pump 55. It is noted that such a data processing device 61 may also optionally be provided to any of the sensor devices 10 described further above, e.g. with respect to Figs. 2 and 3.

### List of reference numerals

- 1: Toilet system
- 2: Bowl
- 3: Seat
- 4: Outlet
- 5: Outlet pipe
- 6: Water seal
- 7: Cistern
- 8: Actuator (flushing device)
- 9: Sensor device (type)
- 10: Sensor device (quantity)
- 11: Chamber
- 12: Outlet of chamber
- 13: Water held back in chamber
- 14: Actuator
- 15: Base
- 16: Water in bowl
- 21: Sensor (for actuating system)
- 22: Weight sensor
- 23: Further sensor
- 24: Analysis device
- 30: Floor
- 31: Box
- 32: Closure mechanism
- 33: Actuator
- 34: Direction of flow in outlet pipe
- 35-39: Opening
- 40: Tip end of opening
- 41: Mounting element
- 51: Flow into chamber
- 52: Screening device
- 53: Weight sensor
- 54: Flow sensor
- 55: Pump
- 56: Pipe
- 57: Inlet
- 58: Valve
- 59: Actuator
- 60: Data processing device/transmitter
- 61: Outlet of valve

- A: Largest size of opening perpendicular to axis L
- B: Largest size of opening parallel to axis L
- C: Shortest distance perpendicular to axis L
- D: Shortest distance parallel to axis L
- L: Longitudinal axis of outlet pipe
- G: Direction of Gravity
- U: Upstream direction
- X: Main axis of opening

## Claims

1. A toilet system (1) comprising a bowl (2) and an outlet (4), the toilet system being adapted for assessing the quantity of faecal matter, when excreted into the bowl by a user of the toilet system, the toilet system further comprising at least one sensor device (10) configured to capture at least one signal indicative of a quantity of excretion excreted into the bowl by a user of the toilet system, the sensor device (10) comprising:
a chamber (11) provided on the outlet (4) of the toilet system in a position downstream of a water seal (6) arranged between the bowl and the outlet (4) of the toilet system, the chamber (11) being arranged adjacent to at least a part of an outlet pipe (5) of the toilet system and being adapted to receive a quantity of liquid pressed through a water seal of the toilet system, and
at least one through opening (35, 36, 37, 38, 39) provided in the outlet of the toilet system in the position downstream of the water seal at which the chamber (11) is provided such as to allow liquid being forced through the water seal to flow into the chamber, wherein
the at least one through opening is arranged with a main axis (X) extending perpendicular to a longitudinal axis (L) of the outlet pipe, **characterised in that**: the at least one through opening comprises a shape tapering in an upstream direction.

2. A toilet system according to claim 1, wherein the at least one through opening comprises a plurality of through openings (35, 36, 37, 38, 39) being provided in the outlet of the toilet system,
wherein the plurality of through openings (35, 36, 37, 38, 39) comprise a largest size A measured in a direction perpendicular to the longitudinal axis of the outlet pipe and a largest size B measured in a direction parallel with the longitudinal axis of the outlet pipe,
wherein neighboring through openings of the plurality of through openings are arranged spaced apart with a shortest distance C measured in a direction perpendicular to the longitudinal axis of the outlet pipe and a shortest distance D measured in a direction parallel with the longitudinal axis of the outlet pipe, and
wherein the plurality of through openings are arranged in a pattern fulfilling the relations C < A and D < 2B.

3. A toilet system according to any one of the above claims, wherein the at least one through opening (35, 36, 37, 38, 39) comprises a shape ending in a tip end pointing in an upstream direction.

4. A toilet system according to any one of the above claims, wherein the at least one through opening (35, 36, 37, 38, 39) comprises a largest size A measured in a direction perpendicular to the longitudinal axis of the outlet pipe and a largest size B measured in a direction parallel with the longitudinal axis of the outlet pipe, and
wherein the largest size A is between 0,5 mm and 100 mm, and/or
wherein the largest size B is between 0,5 mm and 100 mm.

5. A toilet system according to any of claim 2 and claims 3-4 when dependent on claim 2, wherein the plurality of through openings (35, 36, 37, 38, 39) comprise at least five openings.

6. A toilet system according to any one of the above claims, wherein the sensor device further comprises any one or more of:
a screening device (52) arranged upstream of the at least one through opening or extending over the at least one through opening, and
a valve (58) mounted in connection with at least one of the one or more openings and configured to control inflow of liquid into the chamber.

7. A toilet system according to any one of the above claims, wherein the sensor device further comprises:
a mass sensor or a weight sensor (53) arranged and configured to monitor a weight of the chamber and to capture at least one signal indicative of a quantity of excretion excreted into the bowl by a user of the toilet system, and/or
a flow sensor (54) arranged and configured to monitor a flow of liquid through the chamber and to capture at least one signal indicative of a quantity of excretion excreted into the bowl by a user of the toilet system.

8. A toilet system according to any one of the above claims, wherein the sensor device further comprises a mounting element (41) adapted for enabling mounting the sensor device on an outlet pipe of a toilet system.

9. A method of providing a toilet system (1) according to any one of the above claims 1 to 9, the toilet system (1) being adapted for assessing the quantity of faecal matter, when excreted into the bowl by a user of the toilet system, the toilet system (1) comprising at least one sensor device (10) configured to capture at least one signal indicative of a quantity of excretion excreted into the bowl by a user of the toilet system, the excretion being in the form of any waste product, solid or liquid, that is eliminated from a human body, and the method comprising the steps of:
- providing at least one through opening (35, 36, 37, 38, 39) in at least a part of a lower half of an outlet (4) of the toilet system in a position downstream of a water seal (6) arranged between the bowl and the outlet of the toilet system such as to allow liquid being forced through the water seal to flow through the at least one through opening and into the chamber, wherein
the at least one through opening is arranged with a main axis (X) extending perpendicular to a longitudinal axis (L) of the outlet pipe, and wherein
the at least one through opening comprises a shape tapering in an upstream direction,
- providing a chamber (11) adapted to receive a quantity of liquid pressed through a water seal of the toilet system, and
- arranging the chamber (11) on the outlet of the toilet system in the position downstream of the water seal (6) arranged between the bowl and the outlet of the toilet system and adjacent to at least a part of a lower half of the outlet pipe of the toilet system.

10. A method according to claim 9, and comprising one or more of the further steps of:
providing a plurality of through openings (35, 36, 37, 38, 39) to comprise a size A measured in a direction perpendicular to the longitudinal axis L of the outlet and a size B measured in a direction parallel with the longitudinal axis L of the outlet, wherein neighboring through openings are arranged spaced apart with a distance C measured in a direction perpendicular to the longitudinal axis L of the outlet and a distance D measured in a direction parallel with the longitudinal axis L of the outlet, and wherein the plurality of openings are arranged in a pattern fulfilling the relations C < A and D < 2B,
providing the at least one through opening or the plurality of through openings (35, 36, 37, 38, 39) to comprise a largest size A measured in a direction perpendicular to the longitudinal axis L of the outlet and a largest size B measured in a direction parallel with the longitudinal axis L of the outlet, where the largest size A is between 0,5 mm and 100 mm, and alternatively, or additionally, where the largest size B is between 0,5 mm and 100 mm, and
providing the plurality of through openings (35, 36, 37, 38, 39) to comprise at least five openings.

## Patentansprüche

1. Toilettensystem (1), umfassend eine Schüssel (2) und einen Auslass (4), wobei das Toilettensystem dazu ausgelegt ist, die Menge von Fäkalien zu bestimmen, wenn diese von einem Benutzer des Toilettensystems in die Schüssel ausgeschieden werden, wobei das Toilettensystem ferner mindestens eine Sensorvorrichtung (10) umfasst, die dazu ausgelegt ist, mindestens ein Signal zu erfassen, das eine Menge von Ausscheidung anzeigt, die von einem Benutzer des Toilettensystems in die Schüssel ausgeschieden wird, wobei die Sensorvorrichtung (10) umfasst:
eine Kammer (11), die am Auslass (4) des Toilettensystems an einer Position stromabwärts eines Wasserverschlusses (6) vorgesehen ist, der zwischen der Schüssel und dem Auslass (4) des Toilettensystems angeordnet ist, wobei die Kammer (11) benachbart zu zumindest einem Teil eines Auslassrohres (5) des Toilettensystems angeordnet ist und dazu ausgelegt ist, eine Menge von Flüssigkeit aufzunehmen, die durch einen Wasserverschluss des Toilettensystems gedrückt wird, und
mindestens eine Durchgangsöffnung (35, 36, 37, 38, 39), die im Auslass des Toilettensystems an der Position stromabwärts des Wasserverschlusses vorgesehen ist, an der die Kammer (11) vorgesehen ist, sodass Flüssigkeit, die durch den Wasserverschluss gedrückt wird, in die Kammer strömen kann, wobei
die mindestens eine Durchgangsöffnung mit einer Hauptachse (X) angeordnet ist, die sich senkrecht zu einer Längsachse (L) des Auslassrohres erstreckt, **dadurch gekennzeichnet, dass**:
die mindestens eine Durchgangsöffnung eine Form aufweist, die sich in stromaufwärtiger Richtung verjüngt.

2. Toilettensystem nach Anspruch 1, wobei die mindestens eine Durchgangsöffnung eine Mehrzahl von Durchgangsöffnungen (35, 36, 37, 38, 39) umfasst, die im Auslass des Toilettensystems vorgesehen sind,
wobei die Mehrzahl von Durchgangsöffnungen (35, 36, 37, 38, 39) eine größte Abmessung A aufweist, gemessen in einer Richtung senkrecht zur Längsachse des Auslassrohres, und eine größte Abmessung B, gemessen in einer Richtung parallel zur Längsachse des Auslassrohres, wobei benachbarte Durchgangsöffnungen der Mehrzahl von Durchgangsöffnungen mit einem kürzesten Abstand C angeordnet sind, gemessen in einer Richtung senkrecht zur Längsachse des Auslassrohres, und einem kürzesten Abstand D, gemessen in einer Richtung parallel zur Längsachse des Auslassrohres, und
wobei die Mehrzahl von Durchgangsöffnungen in einem Muster angeordnet ist, das die Beziehungen C < A und D < 2B erfüllt.

3. Toilettensystem nach einem der vorstehenden Ansprüche, wobei die mindestens eine Durchgangsöffnung (35, 36, 37, 38, 39) eine Form aufweist, die in einem Spitzenende endet, das in stromaufwärtiger Richtung weist.

4. Toilettensystem nach einem der vorstehenden Ansprüche, wobei die mindestens eine Durchgangsöffnung (35, 36, 37, 38, 39) eine größte Abmessung A aufweist, gemessen in einer Richtung senkrecht zur Längsachse des Auslassrohres, und eine größte Abmessung B, gemessen in einer Richtung parallel zur Längsachse des Auslassrohres, und
wobei die größte Abmessung A zwischen 0,5 mm und 100 mm liegt, und/oder
wobei die größte Abmessung B zwischen 0,5 mm und 100 mm liegt.

5. Toilettensystem nach Anspruch 2 oder einem der Ansprüche 3-4, wenn abhängig von Anspruch 2, wobei die Mehrzahl von Durchgangsöffnungen (35, 36, 37, 38, 39) mindestens fünf Öffnungen umfasst.

6. Toilettensystem nach einem der vorstehenden Ansprüche, wobei die Sensorvorrichtung ferner eines oder mehrere der folgenden Elemente umfasst:
eine Abschirmvorrichtung (52), die stromaufwärts der mindestens einen Durchgangsöffnung angeordnet ist oder sich über die mindestens eine Durchgangsöffnung erstreckt, und
ein Ventil (58), das in Verbindung mit mindestens einer der einen oder mehreren Öffnungen montiert ist und dazu ausgelegt ist, einen Zufluss von Flüssigkeit in die Kammer zu steuern.

7. Toilettensystem nach einem der vorstehenden Ansprüche, wobei die Sensorvorrichtung ferner umfasst:
einen Massensensor oder einen Gewichtssensor (53), der angeordnet und dazu ausgelegt ist, ein Gewicht der Kammer zu überwachen und mindestens ein Signal zu erfassen, das eine Menge von Ausscheidung anzeigt, die von einem Benutzer des Toilettensystems in die Schüssel ausgeschieden wird, und/oder
einen Durchflusssensor (54), der angeordnet und dazu ausgelegt ist, einen Flüssigkeitsfluss durch die Kammer zu überwachen und mindestens ein Signal zu erfassen, das eine Menge von Ausscheidung anzeigt, die von einem Benutzer des Toilettensystems in die Schüssel ausgeschieden wird.

8. Toilettensystem nach einem der vorstehenden Ansprüche, wobei die Sensorvorrichtung ferner ein Montageelement (41) umfasst, das dazu ausgelegt ist, das Montieren der Sensorvorrichtung an einem Auslassrohr eines Toilettensystems zu ermöglichen.

9. Verfahren zum Bereitstellen eines Toilettensystems (1) nach einem der vorstehenden Ansprüche 1 bis 9, wobei das Toilettensystem (1) dazu ausgelegt ist, die Menge von Fäkalien zu bestimmen, wenn diese von einem Benutzer des Toilettensystems in die Schüssel ausgeschieden werden, wobei das Toilettensystem (1) mindestens eine Sensorvorrichtung (10) umfasst, die dazu ausgelegt ist, mindestens ein Signal zu erfassen, das eine Menge von Ausscheidung anzeigt, die von einem Benutzer des Toilettensystems in die Schüssel ausgeschieden wird, wobei die Ausscheidung in Form eines beliebigen Abfallprodukts vorliegt, fest oder flüssig, das aus einem menschlichen Körper ausgeschieden wird, und wobei das Verfahren die folgenden Schritte umfasst:
- Bereitstellen von mindestens einer Durchgangsöffnung (35, 36, 37, 38, 39) in zumindest einem Teil einer unteren Hälfte eines Auslasses (4) des Toilettensystems an einer Position stromabwärts eines Wasserverschlusses (6), der zwischen der Schüssel und dem Auslass des Toilettensystems angeordnet ist, sodass Flüssigkeit, die durch den Wasserverschluss gedrückt wird, durch die mindestens eine Durchgangsöffnung und in die Kammer strömen kann, wobei
die mindestens eine Durchgangsöffnung mit einer Hauptachse (X) angeordnet ist, die sich senkrecht zu einer Längsachse (L) des Auslassrohres erstreckt, und wobei
die mindestens eine Durchgangsöffnung eine Form aufweist, die sich in stromaufwärtiger Richtung verjüngt,
- Bereitstellen einer Kammer (11), die dazu ausgelegt ist, eine Menge von Flüssigkeit aufzunehmen, die durch einen Wasserverschluss des Toilettensystems gedrückt wird, und
- Anordnen der Kammer (11) am Auslass des Toilettensystems an der Position stromabwärts des Wasserverschlusses (6), der zwischen der Schüssel und dem Auslass des Toilettensystems angeordnet ist, und benachbart zu zumindest einem Teil einer unteren Hälfte des Auslassrohres des Toilettensystems.

10. Verfahren nach Anspruch 9, ferner umfassend einen oder mehrere der folgenden weiteren Schritte:
Bereitstellen einer Mehrzahl von Durchgangsöffnungen (35, 36, 37, 38, 39), sodass diese eine Abmessung A aufweisen, gemessen in einer Richtung senkrecht zur Längsachse L des Auslasses, und eine Abmessung B, gemessen in einer Richtung parallel zur Längsachse L des Auslasses, wobei benachbarte Durchgangsöffnungen mit einem Abstand C angeordnet sind, gemessen in einer Richtung senkrecht zur Längsachse L des Auslasses, und einem Abstand D, gemessen in einer Richtung parallel zur Längsachse L des Auslasses, und wobei die Mehrzahl von Öffnungen in einem Muster angeordnet ist, das die Beziehungen C < A und D < 2B erfüllt,
Bereitstellen der mindestens einen Durchgangsöffnung oder der Mehrzahl von Durchgangsöffnungen (35, 36, 37, 38, 39), sodass diese eine größte Abmessung A aufweisen, gemessen in einer Richtung senkrecht zur Längsachse L des Auslasses, und eine größte Abmessung B, gemessen in einer Richtung parallel zur Längsachse L des Auslasses, wobei die größte Abmessung A zwischen 0,5 mm und 100 mm liegt, und alternativ oder zusätzlich, wobei die größte Abmessung B zwischen 0,5 mm und 100 mm liegt, und
Bereitstellen der Mehrzahl von Durchgangsöffnungen (35, 36, 37, 38, 39), sodass diese mindestens fünf Öffnungen umfasst.

## Revendications

1. Système de toilettes (1) comprenant une cuvette (2) et une sortie (4), le système de toilettes étant conçu pour évaluer la quantité de matières fécales, lorsqu'excrétées dans la cuvette par un utilisateur du système de toilettes, le système de toilettes comprenant en outre au moins un dispositif capteur (10) configuré pour capturer au moins un signal indicatif d'une quantité d'excrétion excrétée dans la cuvette par un utilisateur du système de toilettes, le dispositif capteur (10) comprenant :
une chambre (11) fournie sur la sortie (4) du système de toilettes dans une position aval d'un joint hydraulique (6) agencé entre la cuvette et la sortie (4) du système de toilettes, la chambre (11) étant agencée adjacente à au moins une partie d'un tuyau de sortie (5) du système de toilettes et étant conçue pour recevoir une quantité de liquide pressé à travers un joint hydraulique du système de toilettes, et
au moins une ouverture traversante (35, 36, 37, 38, 39) fournie dans la sortie du système de toilettes dans la position aval du joint hydraulique au niveau de laquelle la chambre (11) est fournie de manière à permettre à du liquide étant forcé à travers le joint hydraulique de s'écouler jusque dans la chambre, dans lequel
l'au moins une ouverture traversante est agencée avec un axe principal (X) s'étendant perpendiculaire à un axe longitudinal (1) du tuyau de sortie, **caractérisé en ce que** :
l'au moins une ouverture traversante comprend un profil s'effilant dans une direction amont.

2. Système de toilettes selon la revendication 1, dans lequel l'au moins une ouverture traversante comprend une pluralité d'ouvertures traversantes (35, 36, 37, 38, 39) étant fournies dans la sortie du système de toilettes, dans lequel la pluralité d'ouvertures traversantes (35, 36, 37, 38, 39) comprend une taille A la plus grande mesurée dans une direction perpendiculaire à l'axe longitudinal du tuyau de sortie et une taille B la plus grande mesurée dans une direction parallèle à l'axe longitudinal du tuyau de sortie,
dans lequel des ouvertures traversantes voisines de la pluralité d'ouvertures traversantes sont agencées espacées d'une distance C la plus courte mesurée dans une direction perpendiculaire à l'axe longitudinal du tuyau de sortie et d'une distance D la plus courte mesurée dans une direction parallèle à l'axe longitudinal du tuyau de sortie, et
dans lequel la pluralité d'ouvertures traversantes sont agencées en un motif obéissant aux relations C < A et D < 2B.

3. Système de toilettes selon l'une quelconque des revendications précédentes, dans lequel l'au moins une ouverture traversante (35, 36, 37, 38, 39) comprend un profil se terminant en une extrémité de pointe dirigée dans une direction amont.

4. Système de toilettes selon l'une quelconque des revendications précédentes, dans lequel l'au moins une ouverture traversante (35, 36, 37, 38, 39) comprend une taille A la plus grande mesurée dans une direction perpendiculaire à l'axe longitudinal du tuyau de sortie et une taille B la plus grande mesurée dans une direction parallèle à l'axe longitudinal du tuyau de sortie, et
dans lequel la taille A la plus grande est comprise entre 0,5 mm et 100 mm, et/ou
dans lequel la taille B la plus grande est comprise entre 0,5 mm et 100 mm.

5. Système de toilettes selon l'une quelconque des revendications 2 et 3 à 4 lorsque dépendantes de la revendication 2, dans lequel la pluralité d'ouvertures traversantes (35, 36, 37, 38, 39) comprennent au moins cinq ouvertures.

6. Système de toilettes selon l'une quelconque des revendications précédentes, dans lequel le dispositif capteur comprend en outre un élément quelconque ou plusieurs parmi :
un dispositif de tamisage (52) agencé en amont de l'ouverture traversante ou s'étendant par-dessus l'au moins une ouverture traversante, et
une soupape (58) montée en raccord avec au moins l'une des une ou plusieurs ouvertures et configurée pour contrôler l'entrée de liquide dans la chambre.

7. Système de toilettes selon l'une quelconque des revendications précédentes, dans lequel le dispositif capteur comprend en outre :
un capteur de masse ou un capteur de poids (53) agencé et configuré pour surveiller un poids de la chambre et pour capturer au moins un signal indicatif d'une quantité d'excrétion excrétée dans la cuvette par un utilisateur du système de toilettes, et/ou
un capteur d'écoulement (54) agencé et configuré pour surveiller un écoulement de liquide à travers la chambre et pour capturer au moins un signal indicatif d'une quantité d'excrétion excrétée dans la cuvette par un utilisateur du système de toilettes.

8. Système de toilettes selon l'une quelconque des revendications précédentes, dans lequel le dispositif capteur comprend en outre un élément de montage (41) conçu pour rendre possible le montage du dispositif capteur sur un tuyau de sortie d'un système de toilettes.

9. Procédé de fourniture d'un système de toilettes (1) selon l'une quelconque des revendications précédentes 1 à 9, le système de toilettes (1) étant conçu pour évaluer la quantité de matières fécales, lorsqu'excrétées dans la cuvette par un utilisateur du système de toilettes, le système de toilettes (1) comprenant au moins un dispositif capteur (10) configuré pour capturer au moins un signal indicatif d'une quantité d'excrétion excrétée dans la cuvette par un utilisateur du système de toilettes, l'excrétion étant sous la forme de tout déchet, solide ou liquide, qui est éliminé d'un corps humain, et le procédé comprenant les étapes consistant à :
- fournir au moins une ouverture traversante (35, 36, 37, 38, 39) dans au moins une partie d'une moitié inférieure d'une sortie (4) du système de toilettes dans une position aval d'un joint hydraulique (6) agencé entre la cuvette et la sortie du système de toilettes de manière à permettre à du liquide étant forcé à travers le joint hydraulique de s'écouler à travers l'au moins une ouverture traversante et jusque dans la chambre, dans lequel
l'au moins une ouverture traversante est agencée avec un axe principal (X) s'étendant perpendiculaire à un axe longitudinal (1) du tuyau de sortie, et dans lequel l'au moins une ouverture traversante comprend un profil s'effilant dans une direction amont,
- fournir une chambre (11) conçue pour recevoir une quantité de liquide pressé à travers un joint hydraulique du système de toilettes, et
- agencer la chambre (11) sur la sortie du système de toilettes dans la position aval du joint hydraulique (6) agencé entre la cuvette et la sortie du système de toilettes et adjacent à au moins une partie d'une moitié inférieure du tuyau de sortie du système de toilettes.

10. Procédé selon la revendication 9, et comprenant une ou plusieurs des étapes supplémentaires consistant à :
fournir une pluralité d'ouvertures traversantes (35, 36, 37, 38, 39) pour comprendre une taille A mesurée dans une direction perpendiculaire à l'axe longitudinal L de la sortie et une taille B mesurée dans une direction parallèle à l'axe longitudinal L de la sortie, dans lequel des ouvertures traversantes voisines sont agencées espacées d'une distance C mesurée dans une direction perpendiculaire à l'axe longitudinal L de la sortie et d'une distance D mesurée dans une direction parallèle à l'axe longitudinal L de la sortie, et dans lequel la pluralité d'ouvertures sont agencées en un motif obéissant aux relations C < A et D < 2B,
fournir l'au moins une ouverture traversante ou la pluralité d'ouvertures traversantes (35, 36, 37, 38, 39) pour comprendre une taille A la plus grande mesurée dans une direction perpendiculaire à l'axe longitudinal L de la sortie et une taille B la plus grande mesurée dans une direction parallèle à l'axe longitudinal L de la sortie, où la taille A la plus grande est comprise entre 0,5 mm et 100 mm, et comme variante, ou de surcroît, où la taille B la plus grande est comprise entre 0,5 mm et 100 mm, et
fournir la pluralité d'ouvertures traversantes (35, 36, 37, 38, 39) pour comprendre au moins cinq ouvertures.
